(19) Europäisches Patentamt
European Patent Office
Office européen des brevets

(11) Veröffentlichungsnummer: **0 171 611**
**B1**

(12) # EUROPÄISCHE PATENTSCHRIFT

(45) Veröffentlichungstag der Patentschrift: **23.09.87**

(51) Int. Cl.⁴: **C 07 C 147/12,** C 07 D 295/12, C 07 C 149/42 // C09B62/503

(21) Anmeldenummer: **85108669.4**

(22) Anmeldetag: **11.07.85**

(54) **Substituierte Phenyloxethylsulfone und Verfahren zu ihrer Herstellung.**

(30) Priorität: **20.07.84 DE 3426729**
**30.01.85 DE 3502991**

(43) Veröffentlichungstag der Anmeldung:
**19.02.86 Patentblatt 86/8**

(45) Bekanntmachung des Hinweises auf die Patenterteilung:
**23.09.87 Patentblatt 87/39**

(84) Benannte Vertragsstaaten:
**CH DE FR GB IT LI**

(56) Entgegenhaltungen:
**DE-A-1 644 663**
**FR-M-6 004**

(73) Patentinhaber: **HOECHST AKTIENGESELLSCHAFT, Postfach 80 03 20, D-6230 Frankfurt am Main 80 (DE)**

(72) Erfinder: **Fuchs, Hermann, Dr., Altenhainer Strasse 2, D-6240 Königstein/Taunus (DE)**
Erfinder: **Papenfuhs, Theodor, Dr., Heinrich-Bleicher- Strasse 40, D-6000 Frankfurt am Main 50 (DE)**
Erfinder: **Brodt, Werner, Dr., Drosselbartweg 43, D-6234 Hattersheim am Main (DE)**
Erfinder: **Kohlhaas, Folker, Dr., Akazienring 64, D-6203 Hochheim am Main (DE)**

**EP 0 171 611 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft neue substituierte Phenyloxethylsulfone der allgemeinen Formel (1)

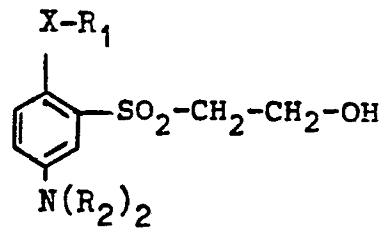

(1)

in welcher X ein Schwefelatom oder die Gruppe

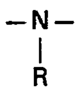

bedeutet, worin R ein Wasserstoffatom, eine Alkylen-$C_1$-$C_4$-alkoxy-$C_1$-$C_6$- oder Alkyl-$C_1$-$C_6$-Gruppe, die durch Hydroxyl-, Sulfonsäure-, Carbonsäure- oder Cyanogruppen substituiert sein können, bedeutet, $R_1$ ein Wasserstoffatom oder eine Alkyl-$C_1$-$C_6$-Gruppe bedeutet, welche durch -$SO_3M$- oder COOM-Gruppen (M bedeutet hierin ein H-, Li-, Na-, K- oder $\frac{Ca}{2}$-Atom), Hydroxyl-, Amino-, Methylamino-, Acetylamino-, Alkyl-$C_1$-$C_4$-sulfonylamino-, Methoxy-, Ethoxy-, β-Hydroxy-ethylsulfonyl-, Phenyl-, Monosulfophenyl-, Disulfophenyl-, 4-[β-Hydroxyethylsulfonyl]-2-sulfo-phenyl-Gruppen substituiert sein kann, oder eine Phenyl- oder Naphthylgruppe darstellt, welche durch -$SO_3M$- oder COOM-Gruppen (M bedeutet hierin ein H-, Li-, Na-, K- oder $\frac{Ca}{2}$-Atom), Alkyl-$C_1$-$C_4$, Alkoxy-$C_1$-$C_4$, Amino-, Methylamino-, Alkyl-$C_1$-$C_4$-sulfonylamino- oder Acetylaminogruppen substituiert sein können, und $R_2$ ein Wasserstoff- oder Sauerstoffatom bedeutet, sowie ein Verfahren zu ihrer Herstellung, indem man Verbindungen der allgemeinen Formel (2)

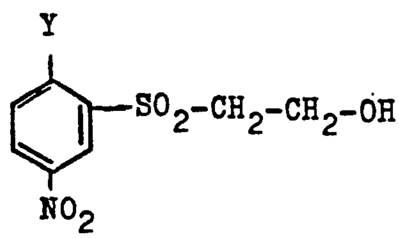

(2)

in welcher Y ein Chlor- oder Bromatom bedeutet, mit einer Verbindung der allgemeinen Formel (3)

H - X - $R_1$ (3)

in welcher X und $R_1$ die vorstehend genannten Bedeutungen haben, in einem hinsichtlich der Reaktanten der genannten Formel (2) und (3) geeigneten Lösungsmittel in Gegenwart eines säurebindenden Mittels bei Temperaturen zwischen 20 und 90°C, vorzugsweise zwischen 40 und 70°C, umsetzt zu Verbindungen der allgemeinen Formel (4)

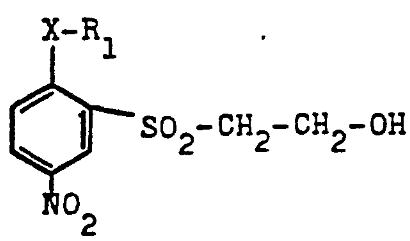

(4)

worin X und $R_1$ die vorstehend genannten Bedeutungen haben, und ggfs. diese in an sich bekannter Weise zu den Verbindungen der genannten Formel (1) mit $R_2 = H$ reduziert.

Als Verbindungen der genannten allgemeinen Formel (3) seien beispielsweise die Verbindungen der folgenden Formeln genannt:

$HS-CH_2-CH_2-OH$
$NH_3$
$H_2N-CH_3$
$H_2N-CH_2-CH_2-CH_2-CH_2-CH_2-CH_3$

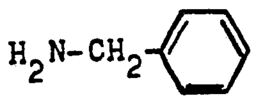

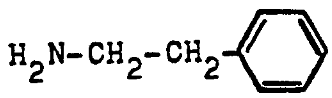

$H_2N-CH_2-CH_2-NH_2$

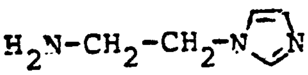

$H_2N-CH_2-CH_2-OH$
$H_2N-CH_2-CH(CH_3)-OH$
$H_2N-CH(CH)_3-CH_2-OH$
$H_2N-CH_2-CH_2-CH_2-CH_2-OH$
$H_2N-CH_2-CH_2-CH_2-CH_2-CH_2-CH_2-NH_2$
$H_2N-CH(C_3H_7)-CH(CH_3)-OH$
$H_2N-CH_2CH_2-O-(CH_2CH_2)_n-OH$ n = 1-3
$H_2N-CH_2-CH_2-OCH_3$
$H_2N-CH_2-CH_2-OC_2H_5$
$H_2N-CH_2-CH_2-NH-CO-CH_3$
$H_2N-CH_2-CH_2-CH_2-NH-CH_3$
$H_2N-CH_2CH_2-NH-(CH_2CH_2-O)_nH$ n = 1-3
$H_2N-CH_2CH_2-N(CH_2CH_2OH)_2$
$H_2N-CH_2-CH_2-SO_2-CH_2-CH_2-OH$
$H_2N-CH_2CH_2-NH-SO_2-CH_2CH_2-OSO_3H$
$H_2N-CH_2-COOH$
$H_2N-CH(CH_3)-COOH$
$H_2N-CH(COOH)-CH_2-SO_3H$
$H_2N-CH_2-CH(SO_3H)-COOH$

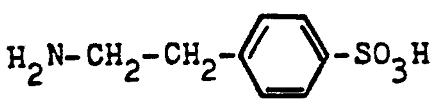

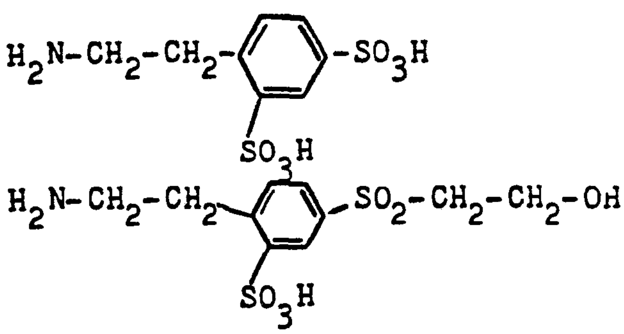

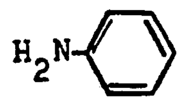

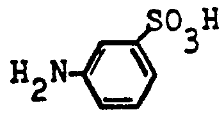

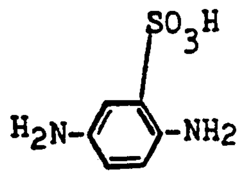

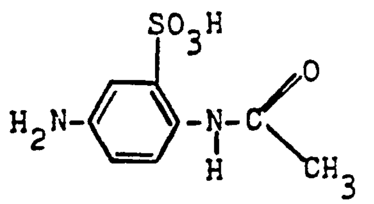

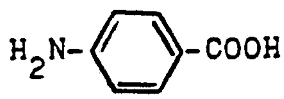

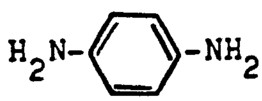

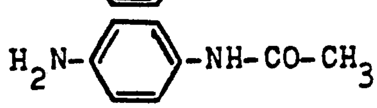

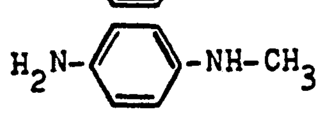

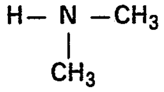

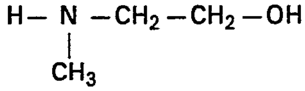

$H-N-CH_2-CH_2-OH$
$\quad\;\; |$
$\quad\;\; C_2H_5$

$H-N-CH_2-CH_2-OH$
$\quad\;\; |$
$\quad\;\; CH_2-CH_2-OH$

$H-N-CH_2-CH_2-CH_2-OH$
$\quad\;\; |$
$\quad\;\; CH_2-CH_2-CH_2-OH$

$H-N-CH_2-CH_2-SO_3H$
$\quad\;\; |$
$\quad\;\; CH_2-CH_2-SO_3H$

$H-N-CH_2-CH_2-SO_3H$
$\quad\;\; |$
$\quad\;\; CH_3$

$H-N-CH_2-COOH$
$\quad\;\; |$
$\quad\;\; CH_2-COOH$

$H-N-CH_2-CH_2-OC_2H_5$
$\quad\;\; |$
$\quad\;\; CH_2-CH_2-O-C_2H_5$

Da bei der ersten Reaktionsstufe Chlor- bzw. Bromwasserstoff abgespalten wird, ist es erforderlich, in Gegenwart eines säurebindenden Mittels zu arbeiten. Hierfür kommen sowohl anorganische Verbindungen, wie beispielsweise Natriumhydroxyd, Naliumhydroxid, Natriumcarbonat, Kaliumcarbonat, Natriumacetat oder Kaliumacetat, als auch organische basische Verbindungen, wie beispielsweise Trialkyl-$C_1$-$C_4$-amine, wie z. B. Trimethyl- oder Triethylamin, oder Pyridin, Chinolin, Picoline und Morpholin in Frage. Das säurebindende Mittel muß jeweils in mindestens äquivalenter Menge zur Monochlor- bzw. Monobromverbindung der Formel (2) vorhanden sein. Stellt der Reaktant der Formel (3) eine basisch reagierende Verbindung, wie beispielsweise Ethanolamin oder Ethylendiamin dar, und setzt man diese in ausreichendem Überschuß ein, so wirkt diese Verbindung als Säureakzeptor, wodurch der Zusatz eines gesonderten anorganischen oder organischen säurebindenden Mittels entfällt.

Als geeignete Lösungsmittel, die als Reaktionsmedium bei der ersten Reaktionsstufe dienen können, kommen beispielsweise in Frage Wasser, Alkanole von 1 bis 4 Kohlenstoffatomen, wie z. B. Methanol, Ethanol, Propanol, Isobutanol, ferner Dioxan, Toluol, Xylole, Chlorbenzol, o-Dichlorbenzol, m-Dichlorbenzol, Dimethylformamid oder N-Methylpyrrolidon. Ist einer der beiden Reaktanten der Formeln (2) und (3) oder beide in dem angewandten Lösungsmittel nicht vollständig löslich, wie beispielsweise in Mercaptoethanol, so findet die Umsetzung teilweise in Suspension statt, was der Umsetzung keinen Abbruch tut.

Es kann jedoch auch der Reaktant der genannten Formel (3) durch Anwendung in einem ausreichenden Überschuß als Lösungsmittel eingesetzt werden.

Bei der Anwendung eines Lösungsmittels in welchem sich beide Reaktanten jeweils vollständig lösen, ist es zweckmäßig, 1 Mol der Verbindung der Formel (2) mit 1-2,5 Mol der Verbindung der Formel (3) zur Umsetzung zu bringen. Die Anwendung eines größeren Überschusses an der Verbindung der Formel (3) ist zwar möglich, bringt aber keinen Vorteil mehr und ist daher nicht zweckmäßig.

Bei Anwendung eines Lösungsmittels, in welchem sich einer oder beide Reaktanten der Formeln (2) und (3) nicht vollständig lösen, oder bei Anwendung von Überschüssigem Reaktant der Formel (3) als Lösungsmittel, ist es zweckmäßig, 1 Mol der Verbindung der Formel (2) mit 1-4 Mol der Verbindung der Formel (3) zur Umsetzung zu bringen. Die Anwendung eines größeren Überschusses an der Verbindung der Formel (3) ist auch hier möglich, aber nicht zweckmäßig.

Aus der Reaktionsmischung kann die Nitroverbindung der Formel (4) in einfachster Weise durch Kristallisation und anschließende Filtration, ggf. nach Abkühlen, isoliert werden. Weiterhin kann die Isolierung in ähnlicher Weise durch Abdestillieren des Lösungsmittels bzw. des überschüssigen Reagens der Formel (3), das dabei vorteilhafterweise für weitere Umsetzungen wiedergewonnen werden kann, erfolgen. Anschließend wird das eingeengte Reaktionsgemisch in eine gekühlte wässrige Vorlage, die ggf. ein Salz, wie beispielsweise NaCl, KCl oder $Na_2SO_4$, enthalten kann, eingerührt. Das dabei auskristallisierende Produkt kann durch Filtration, ggf. nach Ansäuern mit Mineralsäure, isoliert werden.

Die so erhaltenen Verbindungen der allgemeinen Formel (4) können anschließend zu den entsprechenden Aminoverbindungen der Formel (1) mit $R_2=H$ reduziert werden. Die Reduktion kann hierbei in an sich bekannter Weise durch katalytische Hydrierung mit Wasserstoff an bekannten Katalysatoren, wie Palladium,

Platin oder Raney-Nickel, bei Temperaturen zwischen 20 und 150°C, vorzugsweise zwischen 50-110°C, und erhöhtem Druck, beispielsweise zwischen 30 und 100 bar, vorzugsweise zwischen 40 und 55 bar, oder durch Reduktion nach Béchamp mit Eisen in saurem oder alkalischem Medium, vorzugsweise in saurem Medium, beispielsweise mit Eisen in Ethanol/Eisessig, erfolgen.

Besonders vorteilhaft wird das erfindungsgemäße Verfahren durchgeführt, indem man die Reaktionslösung der Chloraustauschreaktion (1. Reaktionsstufe) ohne Zwischenisolierung der Verbindungen der Formel (4) direkt der katalytischen Hydrierung unterwirft. Auf diese Weise können Energie- und Abwasserkosten gesenkt werden.

Die Reduktion, sei es die katalytische oder die nach Béchamp, wird zweckmäßigerweise in einem geeigneten Lösungsmitel, wie Wasser, Methanol oder Ethanol, oder in einem Gemisch aus Wasser und Methanol bzw. Wasser und Ethanol durchgeführt, weil sich hierbei die Zielprodukte (Verbindungen der Formel (1) mit $R_2=H$) in einfacher Weise durch Abkühlen, ggf. nach Ansäuern mit Mineralsäure, als freie Aminoverbindung oder in Form des mineralsauren Salzes zur Kristallisation bringen lassen und durch nachfolgende Filtration isoliert werden können.

Die Mutterlaugen können für nachfolgende Hydrieransätze im Kreislauf geführt werden. Ebenso läßt sich aus der Mutterlauge das organische Lösungsmittel durch einfache Destillation bei Normaldruck zurückgewinnen.

Nach dem erfindungsgemäßen Verfahren ist es möglich, die neuen Verbindungen der Formel (1) in guten bis sehr guten Ausbeuten zu gewinnen. Ferner können erfindungsgemäß die Verbindungen der Formel (1) mit $R_2=O$ durchweg, mit $R_2=H$ soweit sie bei der Reduktion kristallin anfallen, in hohem Reinhaltsgrad erhalten werden, wobei außerdem das Abwasser nur in geringem Maße mit anorganischen Salzen belastet wird.

Die erfindungsgemäß erhältlichen neuen Verbindungen der genannten allgemeinen Formel (1) stellen wertvolle Zwischenprodukte zur Herstellung faserreaktiver Azo- und Dioxazinfarbstoffe dar. Zur Herstellung dieser Farbstoffe setzt man zunächst die Verbindungen der allgemeinen Formel (1) ($R_2=H$) mit Chloranil um, wobei man nach oxidativem Ringschluß und nachfolgender Veresterung mit Oleum Farbstoffe der Formel

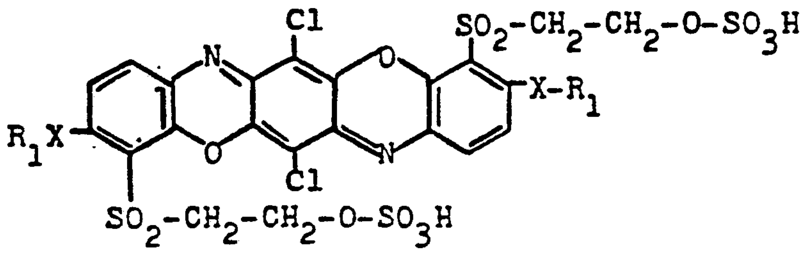

erhält. Ein Farbstoff dieser allgemeinen Formel mit $-XR_1$ = $-NH-CH_2-CH_2-OSO_3H$ ergibt auf Baumwolle reine blaue Färbungen.

**Beispiel 1**

a) (2-β-Hydroxyethylamino-5-nitrophenyl-oxethylsulfon)

Zu einer Lösung von 76,5 g Ethanolamin in 500 ml Methanol gibt man bei 65°C 132,85 g 2-Chlor-5-nitrophenyl-oxethyl-sulfon in 15 Minuten zu und rührt 6 Stunden bei dieser Temperatur. Nach beendeter Reaktion werden unter kontinuierlichem Zulauf von Wasser 400 ml Methanol abdestilliert. Beim Abkühlen der Reaktionslösung kristallisiert das Produkt aus und wird durch Filtration isoliert; das abdestillierte Lösungsmittel wird für Folgereaktionen eingesetzt. Man erhält 135 g 2-β-Hydroxyethylamino-5-nitrophenyl-oxethylsulfon (Reingehalt 99 %) als gelben Feststoff vom Schmelzpunkt 130-132°C, was einer Ausbeute von 93 % der Theorie, bezogen auf eingesetztes 2-Chlor-5-nitrophenyl-oxethylsulfon entspricht.

b) (5-Amino-2-β-hydroxyethylaminophenyl-oxethylsulfon)

Für die katalytische Hydrierung des gemäß Absatz a) erhaltenen 2-β-Hydroxyethylamino-5-nitrophenyl-oxethylsulfons werden 116 g dieses Produktes in 800 ml Methanol bei 100°C und 50 bar Wasserstoff an einem Nickelkatalysator hydriert. Nach Abfiltrieren des Katalysators wird das Produkt durch Abkühlen zur Kristallisation gebracht und durch Filtration isoliert. Man erhält 90,4 g 5-Amino-2-β-hydroxyethylaminophenyl-oxethylsulfon vom Schmelzpunkt 136-138°C mit einem Reingehalt von > 96 %, was einer Ausbeute von 83,5 % der Theorie, bezogen auf eingesetztes 2-β-Hydroxyethylamino-5-nitrophenyl-oxethylsulfon entspricht.

**Beispiel 2**

Wiederholt man Beispiel 1, ohne jedoch nach dem 1. Reaktionsschritt das Produkt zu isolieren, so erhält man nach katalytischer Hydrierung unter den Bedingungen von Beispiel 1b) 109,3 g 5-Amino-2-β-hydroxyethylaminophenyl-oxethylsulfon vom Schmelzpunkt 135-138°C, was einer Ausbeute von 84 % der

Theorie, bezogen auf eingesetztes 2-Chlor-5-nitrophenyl-oxethylsulfon, entspricht.

**Beispiel 3**

a) (2-β-Aminoethylamino-5-nitrophenyl-oxethylsulfon)
Zu 250 g 1,2-Diaminoethan werden bei 65-70°C unter Rühren 132,85 g 2-Chlor-5-nitrophenyl-oxethylsulfon in 15 Minuten eingetragen und weitere 15 Minuten bei dieser Temperatur gerührt. Anschließend läßt man unter Rühren auf Raumtemperatur abkühlen, gießt das Reaktionsgemisch auf 1 l Eiswasser und isoliert das Produkt durch Filtration. Man erhält 139,6 g (2-β-Aminoethylamino)-5-nitrophenyl-oxethylsulfon vom Schmelzpunkt 147°C mit einem Reingehalt von > 98 %, was einer Ausbeute von 94,5 % der Theorie, bezogen auf eingesetztes 2-Chlor-5-nitrophenyl-oxethylsulfon, entspricht.

b) (5-Amino-2-β-aminoethylaminophenyl-oxethylsulfon)
Hydriert man die gemäß Absatz a) gewonnene Verbindung katalytisch gemäß Beispiel 7b), so erhält man 77,5 g 5-Amino-2-β-aminoethylaminophenyl-oxethylsulfon. Durch Behandeln mit methanolischer Salzsäure läßt sich das Produkt als Hydrochlorid vom Schmelzpunkt 242°C kristallin isolieren.

**Beispiel 4**

(2-β-Hydroxyethylmercapto-5-nitrophenyl-oxethylsulfon)
Zur Suspension von 132,85 g 2-Chlor-5-nitrophenyl-oxethylsulfon in 100 ml Wasser und 80 g 2-Mercaptoethanol werden bei 22°C 58,75 g Kaliumcarbonat im Verlaufe von 1,5 Stunden zugegeben und 4 Stunden bei Raumtemperatur nachgerührt. Anschließend saugt man das Reaktionsgemisch ab und wäscht mit Wasser neutral. Nach dem Trocknen erhält man 124,3 g 2-β-Hydroxyethylmercapto-5-nitrophenyl-oxethylsulfon vom Schmelzpunkt 116-118°C (Reingehalt ca. 99 %), was einer Ausbeute von 81 % der Theorie, bezogen auf eingesetztes 2-Chlor-5-nitrophenyl-oxethylsulfon, entspricht.

**Beispiel 5**

a) (2-Phenethylamino-5-nitrophenyl-oxethylsulfon)
Zu der Lösung von 151,3 g Phenethylamin in 500 ml iso-Propanol gibt man 132,85 g 2-Chlor-5-nitrophenyl-oxethylsulfon bei 60°C zu und heizt anschließend auf 84°C. Man rührt 1 Stunde bei dieser Temperatur und läßt dann abkühlen. Das Produkt wird durch Eingießen der Reaktionslösung in Eiswasser kristallisiert und durch Filtration isoliert. Man erhält 163,1 g 2-Phenethylamino-5-nitrophenyl-oxethylsulfon vom Schmelzpunkt 95-95,5°C und einem Reingehalt von 99,5 %. Dies entspricht einer Ausbeute von 93 % der Theorie, bezogen auf eingesetztes 2-Chlor-5-nitrophenyl-oxethylsulfon.

b) (5-Amino-2-phenethylaminophenyl-oxethylsulfon)
Hydriert man die gemäß Absatz a) erhaltene Verbindung katalytisch gemäß Beispiel 7b), so erhält man 162.0 g 5-Amino-2-phenethylaminopnenyl-oxethylsulfon als Hydrochlorid vom Schmelzpunkt 197°C. Der Reingehalt beträgt 98 %, entsprechend einer Ausbeute von 89 % der Theorie, bezogen auf eingesetztes 2-Phenethylamino-5-nitrophenyl-oxethylsulfon.

**Beispiel 6**

(2-(4-Aminophenylamino)-5-nitrophenyl-oxethylsulfon)
Zu der Lösung von 118,8 g 1,4-Phenylendiamin in 500 ml Methanol werden bei 65°C unter Stickstoffatmosphäre 132,85 g 2-Chlor-5-nitrophenyl-oxethylsulfon in 30 Minuten eingetragen. Man rührt noch 4 Stunden bei dieser Temperatur nach, läßt auf Raumtemperatur abkühlen und kristallisiert das Reaktionsprodukt durch Zugabe von 1 l Eiswasser. Nach Absaugen und Trocknen erhält man 168 g 2-(4-Aminophenyl-amino)-5-nitrophenyl-oxethylsulfon vom Schmelzpunkt 147-149°C mit einem Reingehalt > 99 %, was einer Ausbeute von 99 % der Theorie, bezogen auf eingesetzes 2-Chlor-5-nitrophenyl-oxethylsulfon, entspricht.

**Beispiel 7**

a) (2-Amino-5-nitrophenyl-oxethylsulfon)
132,85 g 2-Chlor-5-nitrophenyl-oxethylsulfon werden in 650 ml i-Propanol mit 41,25 g gasförmigem Ammoniak in einem Autoklav 2 Stunden auf 130°C erhitzt. Nach dem Abkühlen auf Raumtemperatur wird auf 1000 ml Wasser gegossen und das bei Kühlung auf 0°C auskristallierende Produkt durch Filtration isoliert. Man erhält 118,2 g 2-Amino-5-nitrophenyl-oxethylsulfon mit einem Schmelzpunkt von 175°C und einer Reinheit von > 99 %, was einer Ausbeute von 94 % der Theorie, bezogen auf eingesetztes 2-Chlor-5-nitrophenyl-oxethylsulfon, entspricht.

b) (2,5-Diaminophenyl-oxethylsulfon)
Die gemäß Absatz a) erhaltene Verbindung wird in 400 ml Methanol gelöst und an einem Palladiumkatalysator bei 60°C und 40 bar Wasserstoff katalytisch hydriert. Nach Abfiltrieren des Katalysators und Abdestillieren des Lösungsmittels erhält man 99,5 g 2,5-Diaminophenyl-oxethylsulfon als dunkelbraunes Öl. Durch Behandeln mit methanolischer Salzsäure kann das Produkt als Hydrochlorid vom Schmelzpunkt 254,5°C kristallin isoliert werden.

**Beispiel 8**

a) (2-Mercaptoethyl-5-nitrophenyl-oxethylsulfon)
132,85 g 2-Chlor-5-nitrophenyl-oxethylsulfon werden zusammen mit 69,1 g Kaliumcarbonat in 500 ml Ethylmercaptan suspendiert und bei 30°C in 2 Stunden umgesetzt. Man destilliert das überschüssige Ethylmercaptan im Hochvakuum ab, nimmt den Rückstand mit Ethanol auf und kristallisiert das Produkt durch Eingießen in Eiswasser. Man erhält nach Filtration 140,5 g 2-Mercaptoethyl-5-nitrophenyl-oxethylsulfon vom Schmelzpunkt 112-114°C (Reingehalt > 95 %), was einer Ausbeute von 91,5 % der Theorie, bezogen auf eingesetztes 2-Chlor-5-nitrophenyl-oxethylsulfon, entspricht.

b) (5-Amino-2-mercaptoethylphenyl-oxethylsulfon)
Hydriert man 118 g der nach Absatz a) gewonnenen Verbindung gemäß Beispiel 7b), so erhält man 92,4 g 5-Amino-2-mercaptoethylphenyl-oxethylsulfon als gelbes Öl. Durch Behandeln mit methanolischer Salzsäure läßt sich das Produkt als Hydrochlorid vom Schmelzpunkt > 330°C kristallin isolieren.

**Beispiel 9**

(5-Amino-2-(methyl-2'-sulfoethyl-)aminophenyl-oxethylsulfon)
Zur Suspension von 201 g N-Methyltaurin-Natriumsalz in 500 ml Methanol werden bei 65°C 132,85 g 2-Chlor-5-nitrophenyl-oxethylsulfon in 15 Minuten zugegeben und 4 Stunden am Rückfluß gerührt. Nach Abkühlen auf Raumtemperatur überführt man die Reaktionsmischung in einen Autoklav und führt bei 40°C und einem Wasserstoffdruck von 40 bar die katalytische Reduktion mit Raney-Nickel durch. Nach Abkühlen auf Raumtemperatur gibt man 102 ml 30,5 %ige Salzsäure zu und kristallisiert das Produkt im Eisbad aus. Nach Absaugen und Trocknen erhält man 142,5 g 5-Amino-2-(methyl-2'-sulfoethyl)-aminophenyl-oxethylsulfon vom Schmelzpunkt 214°C (Zersetzung) mit einem Reingehalt von 94 %, was einer Ausbeute von 89,5 % der Theorie, bezogen auf eingesetztes 2-Chlor-5-nitrophenyl-oxethylsulfon, entspricht.

**Beispiel 10**

a) (2-γ-Hydroxypropylamino-5-nitrophenyl-oxethylsulfon)
Zu einer Lösung von 93,5 g 3-Aminopropanol-1 in 500 ml Methanol gibt man bei 65°C 132,85 g 2-Chlor-5-nitrophenyl-oxethylsulfon in 15 Minuten zu, rührt 3 Stunden bei dieser Temperatur nach und destilliert das Lösungsmittel unter gleichzeitigem Zulauf von 400 ml Wasser weitgehend ab, kühlt auf 0°C ab und isoliert das dabei auskristallisierende Produkt durch Filtration. Man erhält 137 g 2-γ-Hydroxy-propylamino-5-nitrophenyl-oxethylsulfon als gelben Feststoff vom Schmelzpunkt 75-77°C mit einer Reinheit 98 %, was einer Ausbeute von 88 % der Theorie, bezogen auf eingesetztes 2-Chlor-5-nitrophenyl-oxethylsulfon, entspricht.

b) (5-Amino-2-γ-hydroxypropylaminophenyl-oxethylsulfon)
Hydriert man die gemäß Absatz a) erhaltene Verbindung gemäß Beispiel 7b), so erhält man 94,1 g 5-Amino-2-(γ-hydroxyphenyl)aminophenyl-oxethylsulfon vom Schmelzpunkt 100-101,5°C mit einer Reinheit 95 %, was einer Ausbeute von 65 % der Theorie, bezogen auf eingesetztes 2-Chlor-5-nitrophenyl-oxethylsulfon, entspricht.

**Beispiel 11**

a) (2-[2-(4-Morpholinyl)-ethyl]-amino-5-nitrophenyl-oxethylsulfon)
Zu einer Lösung von 143,3 g 2-(4-Morpholinyl)-ethylamin in 500 ml iso-Propanol gibt man bei 50° C 132,85 g 2-Chlor-5-nitrophenyl-oxethylsulfon in 15 Minuten zu und rührt anschließend 2 Stunden bei 70° C. Nach Abkühlen wird die Reaktionsmischung auf 1500 ml Eiswasser gegossen. Dabei kristallisiert das Produkt aus und wird durch Filtration isoliert. Man erhält 169,8 g 2-[2-(4-Morpholinyl)-ethyl]-amino-5-nitrophenyl-oxethylsulfon vom Schmelzpunkt 100-101° C und einem Reingehalt > 99 5 % (HPLC), was einer Ausbeute von 94 % der Theorie, bezogen auf eingesetztes 2-Chlor-5-nitrophenyl-oxethylsulfon, entspricht.

b) (5-Amino-2-[2-(4-morpholinyl)-ethyl]-amino-phenyl-oxethylsulfon
Die gemäß Absatz a) erhaltene Verbindung wird in 1000 ml Methanol bei 40° C und 50 bar Wasserstoff katalytisch hydriert. Nach Abfiltrieren des Katalysators und Abdestillieren des Lösungsmittels erhält man 157,7 g 5-Amino-2-[2-(4-morpholinyl)-ethyl]-amino-phenyloxethylsulfon als dunkelbraunes Öl mit einem Reingehalt von 99,5 % (HPLC), entsprechend einer Ausbeute von 95,5 %, bezogen auf eingesetzte Nitroverbindung.
MS [DCI ($NH_3$)] : m/z = 347 (M + $NH_4^+$)

**Beispiel 12**

a) (2-Morpholinyl-5-nitrophenyl-oxethylsulfon)
Zu einer Lösung von 108 g Morpholin in 500 ml iso-Butanol gibt man bei 60° C 132,85 g 2-Chlor-5-nitrophenyl-oxethylsulfon in 25 Minuten zu und rührt danach 1 Stunde bei 75° C. Man läßt Abkühlen und gießt das Reaktionsgemisch auf 1500 ml Eiswasser. Dabei kristallisiert das Reaktionsprodukt aus und wird durch Filtration isoliert.
Man erhält 152 g 2-Morpholinyl-5-nitrophenyl-oxethylsulfon mit einem Schmelzpunkt von 155-6° C und einer Reinheit > 99,7 % (HPLC), was einer Ausbeute von 96 % der Theorie, bezogen auf eingesetztes 2-Chlor-5-nitrophenyl-oxethylsulfon entspricht.
MS: [DCI ($NH_3$)] : m/z = 317 ($MH^+$)

b) (5-Amino-2-morpholinyl-phenyloxethylsulfon)
Die gemäß Absatz a) erhaltene Verbindung wird in 500 ml Methanol gelöst und bei 85° C und 50 bar Wasserstoff katalytisch hydriert. Nach Abfiltrieren des Katalysators von der noch heißen Reaktionslösung läßt man das Produkt im Eisbad auskristallisieren. Man erhält 123 g 5-Amino-2-morpholinylphenyloxethylsulfon vom Schmelzpunkt 203-204° C. Der Reingehalt beträgt 99 % (HPLC), entsprechend einer Ausbeute von 85,1 % der Theorie, bezogen auf eingesetztes 2-Morpholinyl-5-nitrophenyl-oxethylsulfon.
MS: [DCI ($NH_3$)] : m/z = 304 (M + $NH_4$(+))

**Beispiel 13**

(2-[2-(2'-Hydroxy-ethoxy)-ethylamino]-5-nitrophenyl-oxethylsulfon)
Zu einer Lösung von 115,5 g 2,2'-Aminoethoxyethanol in 500 ml Methanol gibt man bei 50° C 132,85 g 2-Chlor-5-nitrophenyl-oxethylsulfon in 20 Minuten zu und rührt danach 4 Stunden bei 65° C. Nach Abkühlen gießt man die Reaktionsmischung auf 1500 ml Eiswasser. Man läßt das Reaktionsprodukt auskristallisieren und filtriert ab. Man erhält 155,3 g 2-[2-(2'-Hydroxy-ethoxy)-ethylamino]-5-nitrophenyl-oxethylsulfon vom Schmelzpunkt 86-87° C mit einer Reinheit von 99 % (HPLC), entsprechend einer Ausbeute von 92 % der Theorie, bezogen auf eingesetztes 2-Chlor-5-nitrophenyl-oxethylsulfon.

| | | | | | |
|---|---|---|---|---|---|
| Elementaranalyse: | Ber: | C 43,1 | H 5,4 | N 8,4 | S 9,6 |
| $C_{12}H_{18}N_2SO_7$ (334,35) | Gef: | C 43,0 | H 5,5 | N 8,5 | S 9,1 |

**Beispiel 14**

a) Anilkondensation
260 g 5-Amino-2-β-hydroxyethylaminophenyl-oxethylsulfon, 57,5 g Magnesiumoxid und 124,1 g 2,3,5,6-Tetrachlor-1,4-benzochinon (Chloranil) werden in 1500 g Wasser aufgeschlämmt, auf 65° C erwärmt und 2-5 Stunden bei 65° C gerührt. Man kühlt auf 20° C und filtriert das dunkelbraune Reaktionsprodukt ab. Nach dem Waschen mit Wasser wird das so erhaltene Anil der Formel

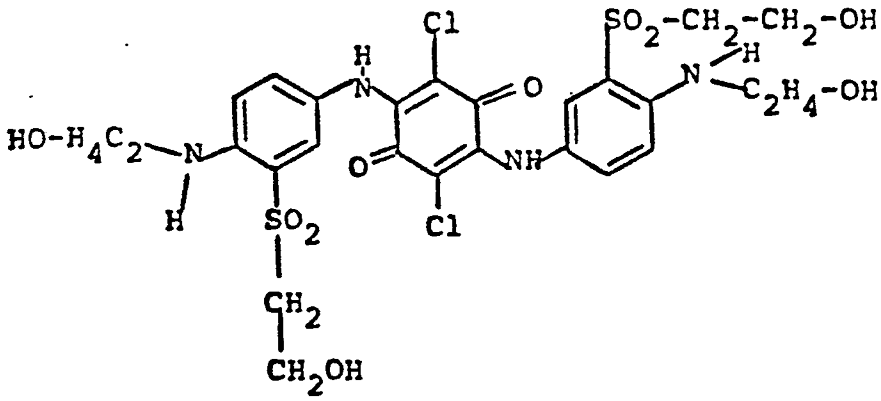

im Vakuum bei 60° C getrocknet.

b) Ringschluß und Veresterung

In 7500 g Oleum 20 % $SO_3$ werden 693 g des wie vorstehend beschrieben erhaltenen Anils so eingetragen, daß die Temperatur bei 18-22° C bleibt. Zum Lösen wird 1 bis 3 Stunden nachgerührt. Dann werden 481 g Natriumperoxodisulfat so eingetragen, daß sich die Temperatur bei 20-23° C hält. Man rührt 15 Stunden nach, gibt auf Eis, stellt mit Calciumcarbonat auf pH 1 bis 1,5 und dann mit Soda auf pH 6. Der entstandene Gips wird abfiltriert und mit Wasser ausgewaschen. Das Filtrat wird eingedampft.

Man erhält einen Farbstoff der Formel

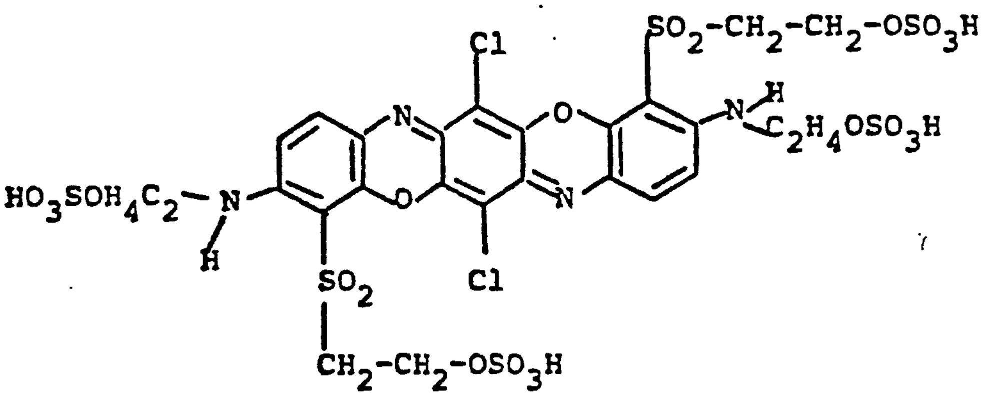

der auf Baumwolle reine blaue Färbungen ergibt.

**Patentansprüche**

1. Verbindungen der allgemeinen Formel (1)

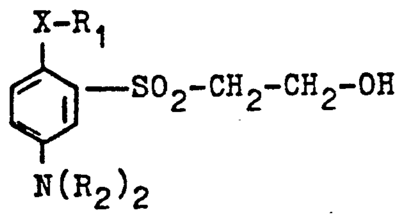

(1)

in welcher X ein Schwefelatom oder die Gruppe

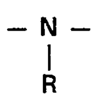

bedeutet, worin R ein Wasserstoffatom, eine Alkylen-$C_1$-$C_4$alkoxy-$C_1$-$C_6$ oder Alkyl-$C_1$-$C_6$Gruppe, die durch

Hydroxyl-, Sulfonsäure-, Carbonsäure- oder Cyanogruppen substituiert sein können, bedeutet, $R_1$ ein Wasserstoffatom oder eine Alkyl-$C_1$-$C_6$Gruppe bedeutet, welche durch -$SO_3M$- oder COOM-Gruppen (M bedeutet hierin ein H-, Li-, Na-, K- oder $\frac{Ca}{2}$-Atom), Hydroxyl-, Amino-, Methylamino-, Acetylamino-, Alkyl-$C_1$-$C_4$sulfonylamino-, Methoxy-, Ethoxy-, β-Hydroxyethylsulfonyl-, Phenyl-, Monosulfophenyl-, Disulfophenyl-, 4-[β-Hydroxyethylsulfonyl]-2-sulfophenyl-Gruppen substituiert sein kann, oder eine Phenyl- oder Naphthylgruppe darstellt, welche durch -$SO_3M$- oder COOM-Gruppen (M bedeutet hierin ein H-, Li-, Na-, K- oder $\frac{Ca}{2}$-Atom), Alkyl-$C_1$-$C_4$, Alkoxy-$C_1$-$C_4$, Amino-, Methylamino-, Alkyl-$C_1$-$C_4$sulfonylamino- oder Acetylaminogruppen substituiert sein können, und $R_2$ ein Wasserstoff- oder Sauerstoffatom darstellt.

2. Verbindung der Formel

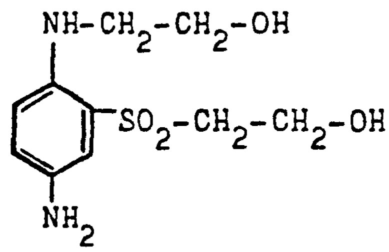

3. Verbindung der Formel

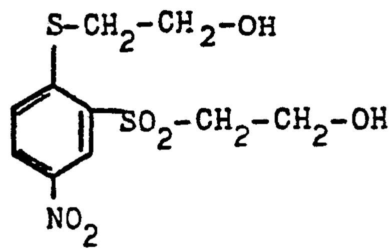

4. Verbindung der Formel

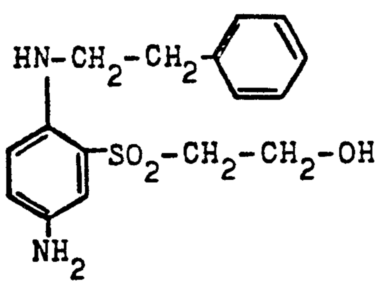

5. Verbindung der Formel

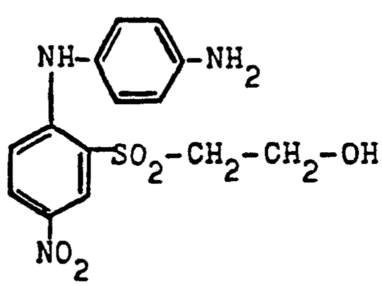

6. Verbindung der Formel

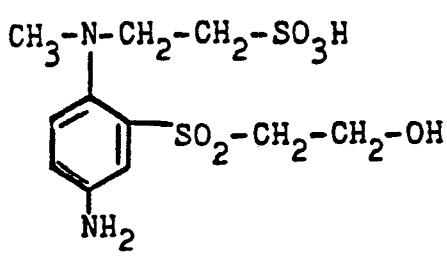

7. Verfahren zur Herstellung von Verbindungen der allgemeinen Formel (1)

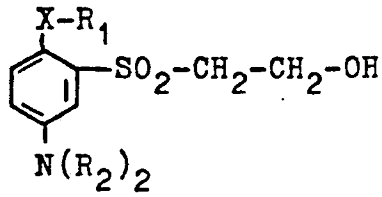

(1)

in welcher X ein Schwefelatom oder die Gruppe

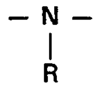

bedeutet, worin R ein Wasserstoffatom, eine Alkylen-$C_1$-$C_4$alkoxy-$C_1$-$C_6$ oder Alkyl-$C_1$-$C_6$-Gruppe, die durch Hydroxyl-, Sulfonsäure-, Carbonsäure- oder Cyanogruppen substituiert sein können, bedeutet, $R_1$ ein Wasserstoffatom oder eine Alkyl-$C_1$-$C_6$Gruppe bedeutet, welche durch $-SO_3M$-oder COOM-Gruppen (M bedeutet hierin ein H-, Li-, Na-, K- oder $\frac{Ca}{2}$-Atom), Hydroxyl-, Amino-, Methylamino-, Acetylamino-, Alkyl-$C_1$-$C_4$sulfonylamino-, Methoxy-, Ethoxy-, β-Hydroxyethylsulfonyl-, Phenyl-, Monosulfophenyl-, DDisulfophenyl-, 4-[β-Hydroxyethylsulfonyl]-2-sulfophenyl-Gruppen substituiert sein kann, oder eine Phenyl- oder Naphthylgruppe darstellt, welche durch $-SO_3M$- oder COOM-Gruppen (M bedeutet hierin ein H-, Li-, Na-, K- oder $\frac{Ca}{2}$-Atom), Alkyl-$C_1$-$C_4$, Alkoxy-$C_1$-$C_4$, Amino-, Methylamino-, Alkyl-$C_1$-$C_4$sulfonylamino- oder Acetylaminogruppen substituiert sein können, und $R_2$ ein Wasserstoff- oder Sauerstoffatom bedeutet, dadurch gekennzeichnet, daß man Verbindungen der allgemeinen Formel (2)

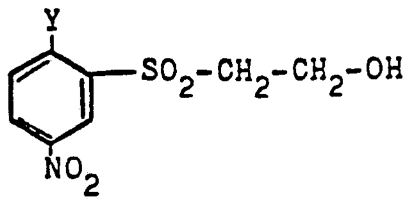

(2)

in welcher Y ein Chlor- oder Bromatom bedeutet, mit einer Verbindung der allgemeinen Formel (3)

$H - X - R_1$ (3)

in welcher X und $R_1$ die vorstehend genannten Bedeutungen haben, in einem hinsichtlich der Reaktanten der genannten Formeln (2) und (3) geeigneten Lösungsmittel in Gegenwart eines säurebindenden Mittels bei Temperaturen zwischen 20 und 90° C umsetzt zu Verbindungen der allgemeinen Formel (4)

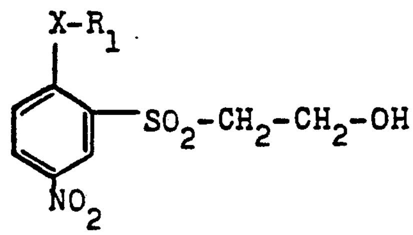

(4)

in welcher X und $R_1$ die vorstehend genannten Bedeutungen haben, und ggfs. diese in an sich bekannter Weise zu den Verbindungen der genannten allgemeinen Formel (1) mit $R_2$ = H reduziert.

8. Verfahren nach Anspruch 7, dadurch gekennzeichnet, daß man die Umsetzung der Verbindungen der allgemeinen Formel (2) mit Verbindungen der allgemeinen Formel (3) in Wasser, Methanol, Ethanol, Propanol, Isobutanol, Dioxan, Toluol, einem Xylol, Chlorbenzol, o-Dichlorbenzol, m-Dichlorbenzol, Dimethylformamid oder N-Methylpyrrolidon als geeignetem Lösungsmittel vornimmt.

9. Verfahren nach Anspruch 7, dadurch gekennzeichnet, daß man die Nitroverbindungen der allgemeinen Formel (4) katalytisch mit Wasserstoff an Palladium, Platin oder Raney-Nickel bei Temperaturen zwischen 20 und 150° C und einem Druck zwischen 30 und 100 bar reduziert.

10. Verfahren nach Anspruch 7, dadurch gekennzeichnet, daß man die Nitroverbindungen der allgemeinen Formel (4) mit Eisen in saurem Medium in Wasser, Methanol, Ethanol, einem Gemisch aus Wasser und Methanol oder einem Gemisch aus Wasser und Ethanol reduziert.

**Claims**

1. Compounds of the general formula (1)

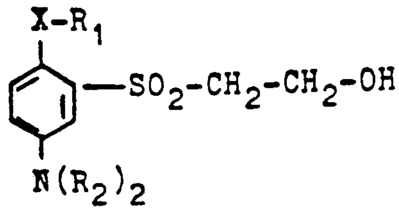

(1)

in which X denotes a sulfur atom or the group

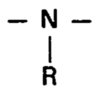

in which R denotes a hydrogen atom, an alkylene-$C_1$-$C_4$-alkoxy-$C_1$-$C_6$- or alkyl-$C_1$-$C_6$-group, each of which can be substituted by hydroxyl, sulfo, carboxyl or cyano groups, $R_1$ denotes a hydrogen atom or an alkyl-$C_1$-$C_6$-group which can be substituted by -$SO_3M$ or COOM groups (where M denotes an H, Li, Na, K or $\frac{Ca}{2}$ atom), hydroxyl, amino, methylamino, acetylamino, alkyl-$C_1$-$C_4$-sulfonylamino, methoxy, ethoxy, β-hydroxyethylsulfonyl, phenyl, monosulfophenyl, disulfophenyl, or 4-[β-hydroxyethylsulfonyl]-2-sulfophenyl groups, or represents a phenyl or naphthyl group, each of which can be substituted by -$SO_3M$ or COOM groups (where M denotes an H, Li, Na, K or $\frac{Ca}{2}$ atom), alkyl-$C_1$-$C_4$-, alkoxy-$C_1$-$C_4$-, amino, methylamino, alkyl-$C_1$-$C_4$-sulfonylamino or acetylamino groups, and $R_2$ represents a hydrogen or oxygen atom.

2. The compound of the formula

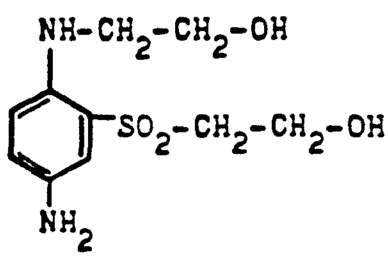

3. The compound of the formula

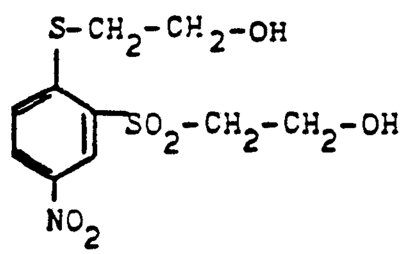

4. The compound of the formula

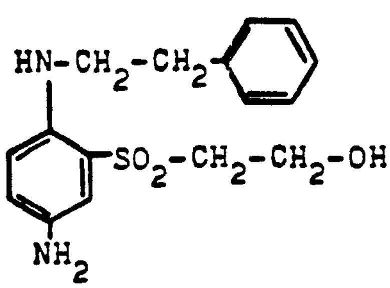

5. The compound of the formula

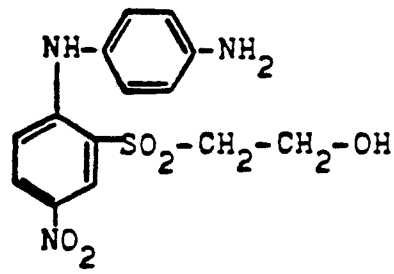

6. The compound of the formula

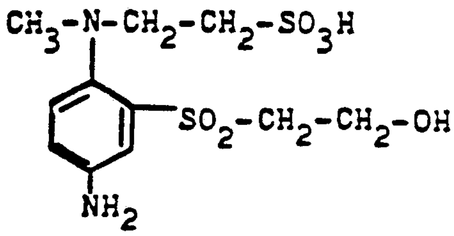

7. A process for preparing compounds of the general formula (1)

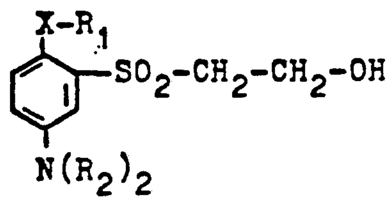 (1)

in which X denotes a sulfur atom or the group

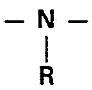

in which R denotes a hydrogen atom, an alkylene-$C_1$-$C_4$-alkoxy-$C_1$-$C_6$- or alkyl-$C_1$-$C_6$- group, each of which can be substituted by hydroxyl, sulfo, carboxyl or cyano groups, $R_1$ denotes a hydrogen atom or an alkyl-$C_1$-$C_6$-group which can be substituted by -$SO_3M$ or COOM groups (where M denotes an H, Li, Na, K or $\frac{Ca}{2}$ atom), hydroxyl, amino, methylamino, acetylamino, alkyl-$C_1$-$C_4$-sulfonyl-amino, methoxy, ethoxy, β-hydroxyethylsulfonyl, phenyl, monosulfophenyl, disulfophenyl, or 4-[β-hydroxyethyl-sulfonyl]-2-sulfophenyl groups, or represents a phenyl or naphthyl group, each of which can be substituted by -$SO_3M$ or COOM groups (where M denotes an H, Li, Na, K or $\frac{Ca}{2}$ atom), alkyl-$C_1$-$C_4$-, alkoxy-$C_1$-$C_4$-, amino, methylamino, alkyl-$C_1$-$C_4$-sulfonylamino or acetylamino groups, and $R_2$ represents a hydrogen or oxygen atom, which comprises reacting compounds of the general formula (2)

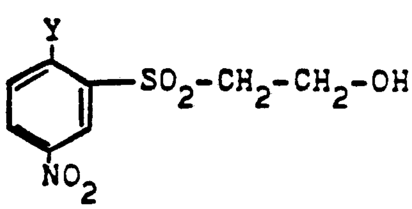 (2)

in which Y denotes a chlorine or bromine atom, with a compound of the general formula (3)
H-X-$R_1$ (3)
in which X and $R_1$ have the abovementioned meanings, at temperatures between 20 and 90°C in a solvent which is suitable in respect of the reactants of the stated formulae (2) and (3) and in the presence of an acid-binding agent to give compounds of the general formula (4)

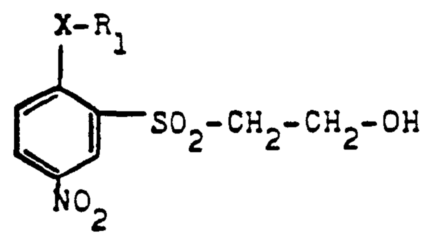

(4)

in which X and $R_1$ have the abovementioned meanings, and if desired reducing these compounds in a manner known per se to the compounds of the stated general formula (1) where $R_2 = H$.

8. The process as claimed in claim 7, wherein the reaction of the compounds of the general formula (2) with compounds of the general formula (3) is carried out in water, methanol, ethanol, propanol, isobutanol, dioxane, toluene, a xylene, chlorobenzene, o-dichlorobenzene, m-dichlorobenzene, dimethylformamide or N-methylpyrrolidone as the suitable solvent.

9. The process as claimed in claim 7, wherein the nitro compounds of the general formula (4) is catalytically reduced at temperatures between 20 and 150°C and a pressure between 30 and 100 bar with hydrogen over palladium, platinum or Raney nickel.

10. The process as claimed in claim 7, wherein the nitro compound of the general formula (4) is reduced with iron in an acid medium in water, methanol, ethanol, a mixture of water and methanol or a mixture of water and ethanol.

**Revendications**

1. Composés de formule générale 1:

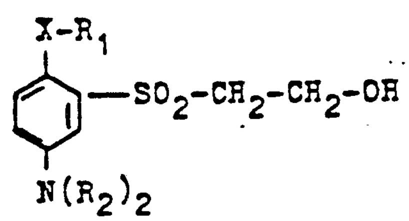

(1)

dans laquelle X représente un atome de soufre ou le groupe

$$\begin{array}{c} -\,N\,- \\ | \\ R \end{array}$$

R étant un atome d'hydrogène ou un groupe alkylène-($C_1$-$C_4$)-alcoxy en $C_1$-$C_6$ ou alkyle en $C_1$-$C_6$ pouvant être substitués par des groupes hydroxyle, sulfonique, carboxylique ou cyano, $R_1$ représente un atome d'hydrogène ou un alkyle en $C_1$-$C_6$ pouvant être substitué par des groupes $-SO_3M$ ou -COOM (M désignant ici un atome H, Li, Na, K ou $\frac{Ca}{2}$), hydroxyle, amino, méthylamino, acétylamino, alkyles($C_1$-$C_4$)-sulfonylamino, methoxy, ethoxy, β-hydroxyéthylsulfonyle, phényle, monosulfophényle, disulfophényle ou 4-[β-hydroxyéthylsulfonyl]-2-sulfophényle, ou encore un groupe phényle ou naphtyle pouvant être substitués par des groupes $-SO_3M$ ou -COOM (M désignant ici un atome H, Li, Na, K ou $\frac{Ca}{2}$), alkyles et alcoxy en $C_1$-$C_4$, amino, méthylamino, alkyles ($C_1$-$C_4$)-sulfonylamino ou acétylamino, et $R_2$ représente un atome d'hydrogène ou d'oxygène.

2. Composé de formule:

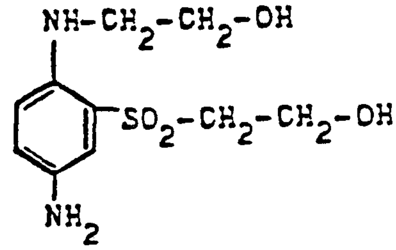

3. Composé de formule:

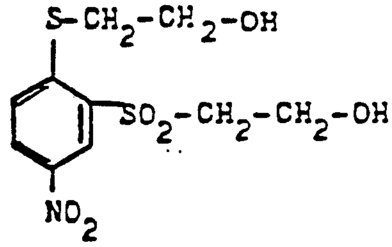

4. Composé de formule:

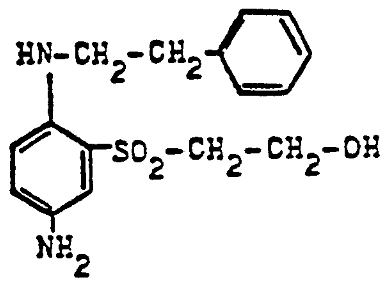

5. Composé de formule:

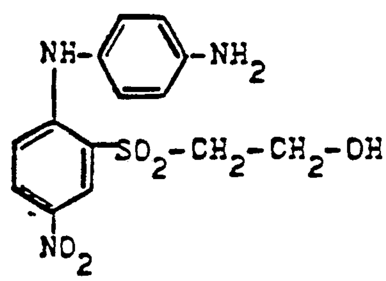

6. Composé de formule:

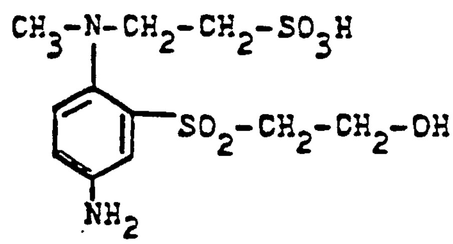

7. Procédé de préparation des composes de formule generale 1:

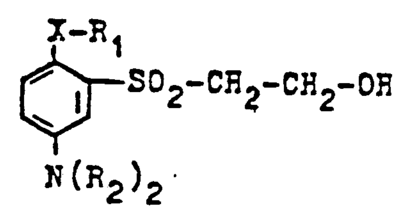 (1)

dans laquelle X représente un atome de soufre ou le groupe

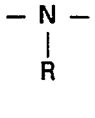

R étant un atome d'hydrogène ou un groupe alkylène-($C_1$-$C_4$)-alcoxy en $C_1$-$C_6$ ou alkyle en $C_1$-$C_6$ pouvant être substitués par des groupes hydroxyle, sulfonique, carboxylique ou cyano, $R_1$ représente un atome d'hydrogène ou un alkyle en $C_1$-$C_6$ pouvant être substitué par des groupes $-SO_3M$ ou -COOM (M désignant ici un atome H, Li, Na, K ou $\frac{Ca}{2}$) hydroxyle, amino, méthylamino, acétylamino, alkyles($C_1$-$C_4$)-sulfonylamino, méthoxy, éthoxy, β-hydroxyéthylsulfonyle, phényle, monosulfophényle, disulfophényle ou 4-[β-hydroxyéthylsulfonyl]-2-sulfophényle, ou encore un groupe phényle ou naphtyle pouvant être substitués par des groupes $-SO_3M$ ou $-COOM$ (M désignant ici un atome H, Li, Na, K ou $\frac{Ca}{2}$), alkyles et alcoxy en $C_1$-$C_4$, amino, méthylamino, alkyles($C_1$-$C_4$)-sulfonylamino ou acétylamino, et $R_2$ représente un atome d'hydrogène ou d'oxygène, procédé caractérisé en ce que l'on fait réagir des composés de formule générale 2:

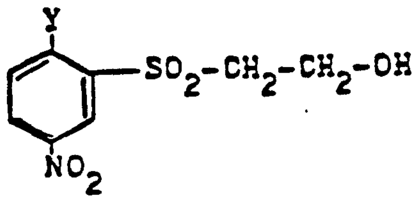
(2)

dans laquelle Y représente un atome de chlore ou de brome, avec un composé de formule générale 3:
$H - X - R_1$ (3)
X et $R_1$ ayant les significations précédentes, dans un solvant approprié aux réactifs de formules 2 et 3 et en présence d'un agent liant les acides, à des températures comprises entre 20 et 90°C, pour former des composés de formule générale 4:

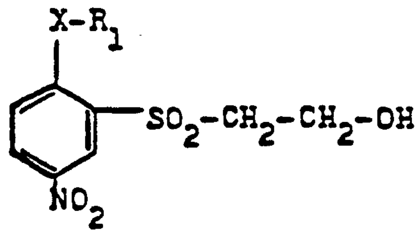
(4)

composés que l'on réduit éventuellement de manière connue en composés de formule 1 dans lesquels $R_2$ est l'hydrogène.

8. Procédé selon la revendication 7 caractérisé en ce que l'on effectue la réaction des composés de formule 2 avec ceux de formule 3 dans de l'eau, du méthanol, de l'éthanol, du propanol, de l'isobutanol, du dioxanne, du toluène, un xylène, du chlorobenzène, de l'ortho-dichlorobenzène, du méta-dichlorobenzène, du diméthylformamide ou de la N-méthylpyrrolidone comme solvant approprié.

9. Procédé selon la revendication 7 caractérisé en ce que l'on réduit les composés nitrés de formule 4 par voie catalytique avec de l'hydrogène sur du palladium, du platine ou du nickel Raney à des températures comprises entre 20 et 150° C et sous une pression de 30 à 100 bar.

10. Procédé selon la revendication 7 caractérisé en ce que l'on réduit les composés nitrés de formule générale 4 avec du fer dans un milieu acide, dans de l'eau, du méthanol, de l'éthanol, un mélange d'eau et de méthanol ou un mélange d'eau et d'éthanol.